# EUROPÄISCHE PATENTSCHRIFT

(11) **EP 0 893 504 B1**
(45) Veröffentlichungstag und Bekanntmachung des Hinweises auf die Patenterteilung: **14.05.2003**
(21) Anmeldenummer: 97111879.9
(22) Anmeldetag: 11.07.1997
(51) Int. Cl.: C12N 15/38, C07K 14/03, A61K 39/245, C12Q 1/70, G01N 33/569, C07K 16/08

(54) **Von Kaposi-sarkom assoziiertem Herpes-Virus (KSHV, HHV-8) kodiertes Polypeptid und dessen Verwendung in Diagnostik und Therapie**
Polypeptide coded by the Kaposi sarcoma-associated virus (KSHV, HHV-8) and its uses in diagnostic and therapy
Polypeptide codé par le virus associé au sarcome de Kaposi (KSHV, HHV-8), et son utilisation en diagnostic et en thérapie.

(43) Veröffentlichungstag der Anmeldung: 27.01.1999
(73) Patentinhaber: Fleckenstein, Bernard, Prof. Dr., 91369 Wiesenthau (DE); Neipel, Frank, Dr., 91080 Uttenreuth (DE); Albrecht, Jens-Christian, 90763 Fürth (DE)
(72) Erfinder: Fleckenstein, Bernhard, Prof. Dr., D-91369 Wiesenthau (DE); Albrecht, Jens-Christian, D-90763 Fürth (DE); Neipel, Frank, Dr., D-91080 Uttenreuth (DE); Lang, Dieter, Dr., D-63128 Dietzenbach (DE)
(74) Vertreter: Keller, Günter, Dr.

(56) Entgegenhaltungen:
- WO-A-96/06159
- EMBL DATENBANK EINTRAG KSU75698; ZUGANGSNUMMER U75698 (VERSION 2), 17. Februar 1997, XP002049355
- RUSSO J J ET AL: "NUCLEOTIDE SEQUENCE OF THE KAPOSI SARCOMA-ASSOCIATED HERPESVIRUS (HHV8)" PROCEEDINGS OF THE NATIONAL ACADEMY OF SCIENCES OF USA, Bd. 93, Nr. 25, 10. Dezember 1996, Seiten 14862-14867, XP002027237

## Beschreibung

Das Kaposi-Sarkom ist ein vasculärer Tumor der Haut, der mittlerweile von großer klinischer und epidemiologischer Bedeutung ist. Ursprünglich trat das Kaposi-Sarkom selten auf, und zwar vorwiegend bei der älteren männlichen Bevölkerung Südund Süd/Ost-Europas (insbesondere Italien und Griechenland).

Mit der Verbreitung des humanen Immundefizienz Virus (HIV) hat das Kaposi-Sarkom wesentlich an Bedeutung gewonnen und stellt heute eine der häufigsten Manifestationen bei AIDS-Patienten dar.

In den letzten Jahren wurde ein Herpes-Virus entdeckt, das zu einem sehr hohen Prozentsatz in Kaposi-Sarkom-Gewebe von AIDS-Patienten gefunden wurde. Dieses Virus wurde als Kaposi-Sarkomassoziiertes Herpes-Virus (KSHV) oder HHV-8 bezeichnet. Von Russo et al. wurde kürzlich die Nucleotidsequenz des Kaposi-Sarkom assoziierten Herpes-Virus publiziert [Proc.Natl.Acad.Sci., USA, Vol. 93 (Dezember 1996), S. 14862-14867].

Die Nucleotidsequenz des KSHV zeigt auffallende Sequenzhomologien zu bekannten Gamma-Herpesviren, wie z.B. dem Herpesvirus Saimiri (HVS), das in Affen vorkommt, und dem Epstein-Barr Virus (EBV). Taxonomisch wird das Virus in die Gruppe der Rhadinoviridae eingereiht.

Das Genom des Virus umfaßt etwa 165 Kilobasen und Russo hat innerhalb dieser Sequenz 81 offene Leserahmen identifiziert, von denen 66 zu offenen Leserahmen des Herpes-Virus Saimiri homolog sind.

Es hat sich herausgestellt, daß das Genom von KSHV auch in AIDS-unabhängigen klassischen und endemischen Formen des Kaposi-Sarkoms nachgewiesen werden kann, sowie auch in anderen seltenen Tumoren, wie dem Body Cavity Based Lymphom (BCBL) und dem Castleman's Disease (CD).

Der Übertragungsmechanismus des KSHV ist noch nicht vollständig aufgeklärt. Da das Virus in Sperma nachgewiesen werden kann, dürfte eine sexuelle Übertragung bei homosexuellen Männern außer Frage stehen. In endemischen Gebieten muß es jedoch noch einen anderen Übertragungsweg geben.

Bisher ist es noch nicht gelungen, Zellen mit KSHV in vitro zuverlässig und mit ausreichender Effizienz zu infizieren. Es gibt B-Zellkultursysteme, z.B. BC-1 und BCBL-1, in denen das Virus latent persistiert und mit einem Phorbolester, nämlich Tetradecanoyl-Phorbol-Acetat (TPA) zum lytischen Replikationszyklus angeregt werden kann.

Für die Erstellung der Genbank verwendeten Russo et al. die Zellinie BC-1, die nicht nur mit dem KSHV, sondern auch mit dem Epstein-Barr Virus koinfiziert ist.

Erfindungsgemäß wurde dagegen die Zellinie BCBL-1 verwendet, die nur mit KSHV infiziert ist. Wenn diese Zellinie mit TPA induziert wird, erfolgt eine KSHV-spezifische Antigenexpression in der Zellkultur und die so exprimierten Antigene wurden zur Bereitstellung der erfindungsgemäßen Polypeptide eingesetzt.

Zunächst wurden die Lysate von induzierten BCBL-1 Zellen untersucht.

Die Zellkultur-abhängigen Westernblot- und Immunfluoreszenz-Analysen vermitteln eine 95 %ige Sensitivität mit den Seren von AIDS-Patienten mit Kaposi-Sarkom. Mittels dieser Analysen konnte gezeigt werden, däß die Seroprävalenz von KSHV in homosexuellen, HIV-positiven Männern signifikant größer ist als in hämophilen HIV-positiven Patienten, so daß man davon ausgehen kann, daß das Virus auf sexuellem Weg übertragen wird. Die Seroprävalenz in der Normalbevölkerung unterliegt starken regionalen Schwankungen. In den USA und Nordeuropa liegt die Seroprävalenz unter 0,5 %, während in Italien 5 % und in Uganda 50 % der Blutproben von gesunden Blutspendern Antikörper gegen KSHV aufweisen. Es konnte auch gezeigt werden, daß etwa 6 bis 75 Monate vor dem Aufreten eines Kaposi-Sarkoms die Serokonversion von KSHV-Antikörpern detektierbar war und prospektiv eine Aussage über die klinische Manifestation von Kaposi-Sarkom möglich war. Daher stellt der Nachweis von KSHV-spezifischen Antikörpern für die meisten Experten einen geeigneten diagnostischen Marker dar. Wegen des erheblichen technischen Aufwands und der mangelnden Standardisierung sind die bestehenden serologischen Testverfahren nur für diagnostische Speziallabors geeignet.

Beschrieben wurde die Bereitstellung eines rekombinanten Kapsidproteins kodiert durch den offenen Leserahmen 65 (ORF65). Es konnte gezeigt werden, daß der C-terminale Teil des Proteins (AS 86-170) gleiche serologische Reaktivität besitzt wie das Protein voller Länge [Simpson et al., Lancet, Vol. 348 (Oktober 1996), S. 11033-11038]. Auch Lin et al. verwenden rekombinantes OR65 Antigen in serologischen Tests zum Nachweis von Antikörpern gegen Kaposi-Sarkom assoziiertem Herpes-Virus. Es handelt sich dabei um das "kleine virale Kapsidantigen" (small viral capsid antigen, sVCA) [Journal of Virology (April 1997), S. 3069-3076], welches das homologe Protein zu BFRF3 von EBV darstellt.

Es ist daher eine Aufgabe der vorliegenden Erfindung, weitere Polypeptide zur Verfügung zu stellen, die in standardisierten und automatisierten Verfahren zum Nachweis von KSHV geeignet sind. Für Immuno Blots oder Enzyme Linked Immuno Sorbent Assays (ELISA) ist die Charakterisierung und Identifizierung von geeigneten immunologisch reaktiven Antigenen erforderlich.

Die erfindungsgemäßen Polypeptide werden durch einen Leserahmen kodiert, der bisher noch nicht identifiziert wurde und erfindungsgemäß als ORF K8.1 bezeichnet wird. Dieser Leserahmen wurde folgendermaßen identifiziert:

In Westernblot-Analysen mit BCBL-1 Zellen, die mit TPA zur lytischen KSHV-Replikation angeregt wurden, konnte mit Seren bestimmter Patienten (HIV-positiv mit Kaposi-Sarkom) eine Proteindoppelbande mit einem Molekulargewicht von etwa 35-37 Kilodalton nachgewiesen werden. Zur Charakterisierung des Proteins (gp35/37) wurden Deglykosilierungsexperimente durchgeführt. Dabei stellte sich heraus, daß die Proteine mit einem Wanderungsverhalten von 35 und 37 kDa in der SDS-Polyacrylamidgelelektrophorese (SDS-PAGE) durch deglykosilierende Enzyme in eine Proteinbande mit 30 kDa überführt werden können. Daraus wurde geschlossen, daß es sich bei dem Protein um ein virales Glykoprotein handeln muß, welches in der mit KSHV infizierten Zelle in zwei Glykosilierungsformen vorliegt.

Da zwischenzeitlich die Sequenz des KSHV aus der BC-1 Zelle ermittelt wurde, wurde diese Sequenz mit unterschiedlichen Suchkriterien zur Identifizierung eines geeigneten offenen Leserahmens durchmustert. Hierzu wurden spezielle Kriterien angewendet, die den üblichen Regeln nicht entsprechen. Zunächst wurde nur nach offenen Leserahmen gesucht, die ein Startkodon (ATG) aufweisen. Es wurde nicht gefordert, daß eine TATA-Box vor dem Transkriptionsstart erforderlich ist. Dadurch konnten offene Leserahmen mit Startkodons gefunden werden, die nicht den üblichen Kozack-Regeln entsprachen. Unter den so aufgefundenen Leserahmen wurden nur solche Leserahmen berücksichtigt, die für ein Protein mit einem Molekulargewicht zwischen 15 und 45 kDa kodieren. Unter den so aufgefundenen Leserahmen wurden weiterhin solche Sequenzen ausgewählt, die eine N-Glykosilierungsstelle aufweisen. Weiterhin wurden die so aufgefundenen potentiellen offenen Leserahmen dadurch eingeschränkt, daß das Vorhandensein eines Signalpeptids gefordert wurde. Von den zunächst aufgefundenen 41 Leserahmen erfüllten fünf die oben genannten Bedingungen, nämlich die offenen Leserahmen ORF47 (Glykoprotein L); K2 (IL6 homolog); ORF70 (Thymidylat Synthase); K1 und K8.1. Im weiteren Verlauf wurde ORF70 nicht weiter berücksichtigt. Die kodierende Sequenz von ORF47, K2, K1 und K8.1 wurde jeweils in den Expressionsvektor pQE9 (von Qiagen) kloniert und die Proteine wurden als Histidin-Fusionsproteine in E.coli exprimiert. Durch den Histidin-Fusionsproteinanteil konnten die Proteine mittels Nickel-Chelat-Agarose gereinigt und in Westernblot-Tests eingesetzt werden. Dabei wurden solche Seren verwendet, die auch mit dem Protein gp35/37 im natürlichen Westernblot reagierten. Während die Proteine der Leserahmen ORF47, K2, K1 keinerlei Reaktivität zeigten, reagierten alle Seren mit dem rekombinanten K8.1 Protein. Eine nachträgliche Überprüfung der Publikation von Russo et al. ergab, daß der erfindungsgemäß verwendete Leserahmen ORF K8.1 nicht angegeben wird.

Um nachzuweisen, daß das Protein gp35/37 im natürlichen Westernblot mit BCBL-1 induzierten Zellen identisch ist mit dem rekombinanten Protein; hergestellt mit Hilfe des erfindungsgemäßen Leserahmens ORF K8.1, wurden Antikörper aus Seren von KS-positiven Patienten mittels präparativer Westernblot-Analyse mit rekombinantem gp35/37 gebunden. Durch anschließende Elution wiedergewonnene Antikörper wurden dann in einem zweiten Westernblot mit TPA induzierten Zellen eingesetzt. Dabei reagierte nur gp35/37 mit den durch rekombinantes Polypeptid K8.1 vorselektionierten Antikörpern. Damit konnte die Übereinstimmung von gp35/37 im natürlichen Immunoblot mit rekombinantem K8.1 gezeigt werden. Die immunogenen und KSHV-spezifischen Eigenschaften des gp35/37 werden unter Verwendung des rekombinanten erfindungsgemäßen Antigens in Immunoblot- und ELISA-Experimenten gezeigt.

Gegenstand der vorliegenden Erfindung sind daher Polypeptide, die dadurch gekennzeichnet sind, daß sie eine Teilsequenz von wenigstens 10 aufeinanderfolgenden Aminosäuren der Sequenz aufweisen.

Unter dem Begriff "Polypeptid" im Sinn der vorliegenden Anmeldung werden Verbindungen verstanden, die wenigstens 10 Aminosäuren umfassen und deren obere Grenze der Aminosäuren bei etwa 250 bis 300 Aminosäuren liegt. Der Begriff umfaßt also auch solche Verbindungen, die auch als Proteine bezeichnet werden können. Oligopeptide, d.h. Verbindungen mit einer sehr geringen Anzahl von Aminosäuren, nämlich weniger als 10 Aminosäuren, werden von dem Begriff nicht umfaßt.

Erfindungsgemäß werden auch Teilsequenzen des Polypeptids K8.1 bevorzugt eingesetzt, und zwar insbesondere solche Teilbereiche des Polypeptids, die antigene Determinanten beinhalten. Das erfindungsgemäße Polypeptid K8.1 besteht aus einem Signalpeptid, das die ersten 26 Aminosäuren (Met ... Ala) umfaßt und dem rekombinanten Polypeptid, d.h. Polypeptid ohne Signalpeptid. Dieses weist 170 Aminosäuren auf, wobei die Sequenz bei Asn beginnt und bei Lys endet.

Besonders bevorzugt als antigene Teilbereiche sind die Polypeptide, die folgende Koordinaten aufweisen:
a) Aminosäure 29-59. Das Polypeptid mit 31 Aminosäuren beginnt mit Pro und endet mit Glu.
b) Polypeptid zwischen den Aminosäuren 73 und 113. Das Polypeptid mit 41 Aminosäuren beginnt mit der Aminosäure Arg und endet mit der Aminosäure Arg.
c) Das Polypeptid beginnt bei Aminosäure 152 und endet bei Aminosäure 196. Dieses Polypeptid mit 45 Aminosäuren beginnt bei der Aminosäure Arg und endet bei der Aminosäure Lys.
d) Aminosäure 183-196. Das Polypeptid mit 14 Aminosäuren beginnt mit Asp und endet mit Lys.

Besonders bevorzugt sind die Polypeptide b) und d), wobei es sich bei dem letztgenannten um die 14 C-terminalen Aminosäuren handelt, die eine stark basische Region darstellen. Auch Kombinationen der Polypeptide a) und b) oder b) und d) sowie von a) und c) sind erfindungsgemäß bevorzugt.

Gegenstand der vorliegenden Erfindung sind auch Varianten der erfindungsgemäßen Polypeptide. Dem Fachmann ist bekannt, daß die Polypeptide an bestimmten Positionen geringfügig verändert werden können, z.B. durch Deletionen oder Austausche von Aminosäuren, wobei insbesondere ein Austausch einer Aminosäure durch eine immunologisch gleichwirkende Aminosäure regelmäßig zu keiner Änderung der immunologischen Aktivität des Polypeptids führt.

Das erfindungsgemäße Polypeptid ohne das Signalpeptid weist folgende Sequenz auf:

Bevorzugte Teilsequenzen des erfindungsgemäßen Polypeptids weisen wenigstens 14, 25, 30 oder besonders bevorzugt 40 Aminosäuren auf, die Fragmente der angegebenen Sequenzen darstellen.

Die erfindungsgemäßen Polypeptide entsprechen hinsichtlich der Sequenz einem Protein, das von einem Kaposi-Sarkom assoziierten Herpes-Virus kodiert wird. Daher reagieren Antikörper eines Patienten, der mit diesem Virus infiziert ist, spezifisch mit den erfindungsgemäßen Polypeptiden. Spezifisch bedeutet, daß eine Antigen-Antikörperreaktion anzeigt, daß eine Infektion mit KSHV vorliegt. Hierzu ist es erforderlich, daß keine Kreuzreaktivität mit einem anderen Virus oder mit anderen Antigenen erfolgt. Eine derartige unspezifische Reaktion würde zu falsch-positiven Ergebnissen führen.

Die erfindungsgemäßen Polypeptide können auf verschiedene Art und Weise hergestellt werden. Für kürzere Polypeptide kann die chemische Synthese gewählt werden. Die chemische Synthese von Polypeptiden ist an sich wohlbekannt und erfolgt üblicherweise an einer Festphase. Nachteilig bei der chemischen Synthese ist jedoch, daß bei längeren Polypeptiden Produkte entstehen, die in der Sequenz nicht mehr korrekt sind, weil falsche Aminosäuren eingebaut wurden und, daß derartige Produkte nur schlecht von den gewünschten Polypeptiden abgetrennt werden können. Außerdem sind chemisch synthetisierte Polypeptide häufig nicht korrekt gefaltet.

Insbesondere längere Polypeptide werden daher nach an sich bekannten Verfahren gentechnologisch in Wirtszellen hergestellt. Hierzu wird die kodierende Nucleinsäure in einen geeigneten Vektor, beispielsweise in ein Expressionsplasmid, eingebaut. Die Expression der erfindungsgemäßen Polypeptide kann dann entweder in prokaryotischen Wirtszellen, beispielsweise E.coli, erfolgen. Diese Produkte weisen jedoch keine Glykosilierung auf, da Bakterien keine Glykosilierungsmechanismen besitzen.

Alternativ hierzu kann die Expression in eukaryotischen Wirtszellen erfolgen. Geeignete Beispiele hierfür wären Hefezellen, Insektenzellen oder aber Säugerzellen, die in Zellkulturen angezogen werden.

Gegenstand der vorliegenden Erfindung sind auch Testkits zum Nachweis einer Infektion mit einem Kaposi-Sarkom assoziierten Herpes-Virus in einem Säugetier, die wenigstens ein erfindungsgemäßes Polypeptid umfassen.

Unter dem Begriff "Testkit" wird eine geeignete Anordnung zum Nachweis von Antikörpern verstanden. Es kann sich hierbei um vorbereitete Sets handeln, die in den diagnostischen Labors zum Nachweis der Antikörper eingesetzt werden können. Bei derartigen Testkits kann es sich beispielsweise um Westernblot-Streifen handeln oder um ELISA-Platten, die bereits mit den erfindungsgemäßen Polypeptiden beschichtet sind. Darüber hinaus kann ein Testkit auch weitere Komponenten umfassen, die zur Durchführung des Tests benötigt werden, wie beispielsweise die Nachweiskomponenten, die zum Nachweis des Polypeptidspezifischer Antikörper-Komplexes dienen.

Eine solche weitere Komponente zum Nachweis des Komplexes umfassend ein erfindungsgemäßes Polypeptid und wenigstens einen hieran spezifisch gebundenen Antikörper kann ein Anti-Antikörper oder ein weiterer Antikörper sein, der gegen ein erfindungsgemäßes Polypeptid gerichtet ist.

Diese Komponente zum Nachweis des Komplexes kann markiert sein, beispielsweise mit einem Enzym, das eine Farbreaktion katalysiert (z.B. Peroxydase). Es kann aber auch das Polypeptid markiert sein. Bei der gentechnischen Herstellung kann das Polypeptid als Fusionsprotein exprimiert werden, wobei mit dem Fusionsanteil eine leichte Markierung oder Abtrennung ermöglicht wird.

Die erfindungsgemäßen Polypeptide werden also bevorzugt zum Nachweis einer Infektion mit einem Kaposi-Sarkom assoziierten Herpes-Virus verwendet.

Gegenstand der Erfindung sind auch Nucleinsäurekonstrukte zur gentechnologischen Herstellung eines Polypeptids, die die Nucleinsäuresequenz oder eine Teilsequenz davon mit wenigstens 30 Nucleotiden umfassen.

Unter dem Begriff "Nucleinsäurekonstrukt" wird erfindungsgemäß ein Nucleinsäuregebilde verstanden, das eine geeignete Nucleinsäuresequenz, kodierend für ein erfindungsgemäßes Polypeptid enthält und, das die Replikation dieses Konstrukts in der Wirtszelle und die Expression des gewünschten Polypeptids in der Wirtszelle ermöglicht. Bei den erfindungsgemäß eingesetzten Nucleinsäuren handelt es sich in bevorzugter Ausführungsform um DNA.

In bevorzugter Ausführungsform ist ein derartiges Nucleinsäurekonstrukt ein Expressionsvektor, d.h. ein Nucleinsäuregebilde, das sich in der eingebrachten Wirtszelle selbst vermehren kann und die Wirtszelle dazu veranlaßt, das erfindungsgemäße Polypeptid zu exprimieren.

In einer weiteren Ausführungsform der Erfindung kann eine Partialsequenz mit 15 bis 35 Nucleotiden aus der Nucleinsäuresequenz oder eine hierzu komplementäre Nucleinsäuresequenz zum Nachweis einer Infektion mit einem Kaposi-Sarkom assoziierten Herpes-Virus mit Hilfe der Polymerase Kettenreaktion verwendet werden.

Beim Nachweisverfahren mit Hilfe der Polymerase Kettenreaktion werden sogenannte Primer, d.h. Nucleinsäurefragmente mit einer Länge von etwa 15 bis etwa 35 Nucleotiden eingesetzt. Diese Primer hybridisieren spezifisch an das nachzuweisende Nucleinsäurefragment. Durch mehrere Amplifikationszyklen wird dann ein bestimmtes DNA-Fragment vermehrt und nachgewiesen. Wenn ein derartiges Fragment nachgewiesen werden kann, bedeutet dies, daß die gesuchte Nucleinsäure (hier von KSHV) in der nachzuweisenden Probe vorhanden ist. Die Primer, die erfindungsgemäß eingesetzt werden können, sind also kurze Nucleinsäurefragmente mit 15 bis 35 Basen Länge. Diese Primer werden bevorzugt am 3'und 5'-Ende der oben näher angegebenen Nucleinsäuresequenz ausgewählt, wobei jeweils ein Primer eine Sequenz hat, die komplementär ist zu der angegebenen Nucleinsäuresequenz.

Die erfindungsgemäßen Polypeptide können auch zur Herstellung eines Impfstoffes gegen das KSHV verwendet werden. Für einen derartigen Impfstoff können die erfindungsgemäßen Polypeptide auch mit anderen Proteinen bzw. Polypeptiden von KSHV kombiniert werden. Durch eine Immunisierung der zu behandelnden Patienten werden neutralisierende Antikörper erzeugt, die bei einer drohenden Infektion das Virus inaktivieren. Eine derartige Impfung ist auch denkbar, wenn der zu behandelnde Patient zwar bereits von dem KSHV infiziert ist, jedoch das Kaposi-Sarkom noch nicht ausgebrochen ist.

Ein weiterer Aspekt der vorliegenden Erfindung betrifft Antikörper, die gegen die erfindungsgemäßen Polypeptide gerichtet sind. Hierbei kann es sich entweder um polyklonale Antikörper handeln, die durch Immunisierung eines Tieres (Pferd, Rind, Ziege) erhalten werden oder um monoklonale Antikörper.

Die Technik der Herstellung von monoklonalen Antikörpern wurde bereits 1974 von Köhler und Millstein beschrieben und stellt derzeit eine Standardtechnologie dar. Hierbei werden Nager (insbesondere Mäuse) immunisiert und die Thymuszellen werden mit immortalisierten Zellen (Krebszellinien) verschmolzen. Diese Zellinien produzieren dann monoklonale Antikörper. Im Rahmen der vorliegenden Erfindung werden jedoch bevorzugt humanisierte monoklonale Antikörper eingesetzt, da bei diesen Antikörpern keine unerwünschten immunologischen Reaktionen gegen die Teile des Antikörpers zu erwarten sind, die nicht vom Menschen abstammen. Hierbei wird mit an sich bekannten gentechnologischen Methoden der für den variablen Bereich (Fab) der Antikörper kodierende Bereich aus den Mäusezellinien übertragen in Zellinien, die für die gewünschten Antikörper kodieren. Dadurch wird ein menschlicher Antikörper erhalten, der in den variablen Regionen Sequenzen beinhaltet, die aus beispielsweise Mäusezellinien stammt.

Im Rahmen der vorliegenden Erfindung wurde völlig überraschend festgestellt, daß die erfindungsgemäßen Polypeptide ganz erhebliche unerwartete Vorteile für die Diagnostik und Therapie aufweisen. Besonders hervorzuheben ist die wesentlich stärkere und häufigere Reaktivität der erfindungsgemäßen Polypeptide mit Seren von Kaposi-Sarkom-Patienten sowie das Fehlen von Kreuzreaktivität. Für die Diagnostik ist es insbesondere wichtig, daß keine Kreuzreaktivität mit anderen, ähnlichen Viren, wie insbesondere EBV vorliegt. Wenn eine Kreuzreaktivität vorliegt, führt dies zu falsch positiven Ergebnissen, weil dann fälschlicherweise Antikörper, die gegen ein anderes Virus (EBV) gerichtet sind, als Antikörper gegen KSHV gewertet werden.

### Erläuterung der Figuren

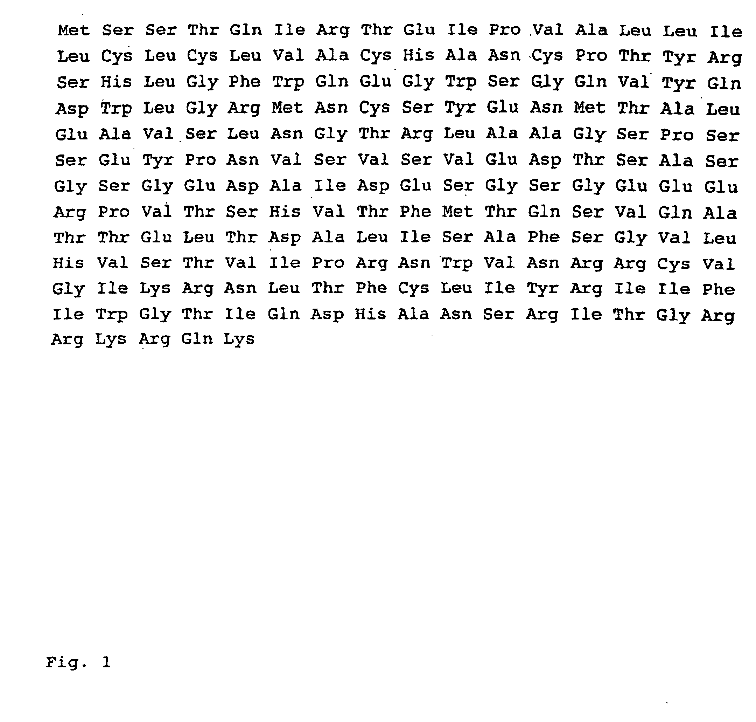

Figur 1 zeigt die Aminosäuresequenz des erfindungsgemäßen Polypepitids K8.1.; die ersten 26 Aminosäuren zwischen den Aminosäuren Met und Ala stellen das Signalpeptid dar. Das erfindungsgemäße Polypeptid umfaßt die Sequenz bis zum Stopkodon, also bis einschließlich Lys.

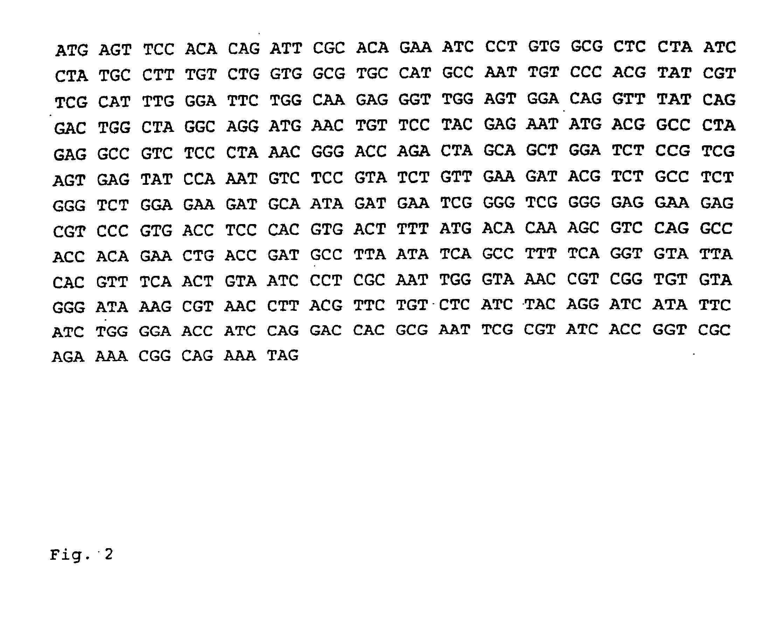

Figur 2 stellt die Nucleotidsequenz dar kodierend für das erfindungsgemäße Polypeptid K8.1 einschließlich Signalpeptid.

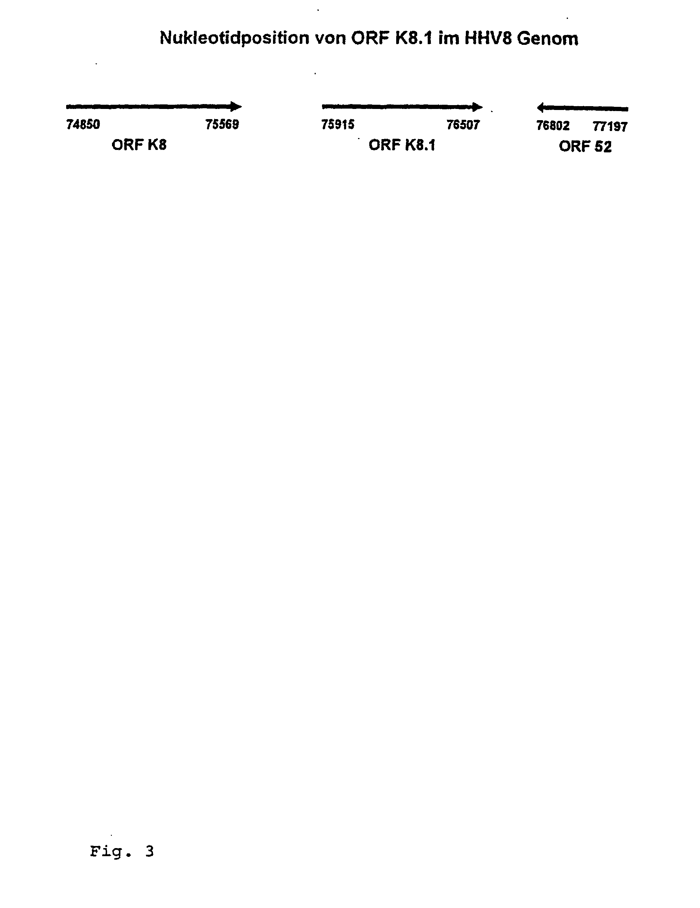

Figur 3 zeigt die Lokalisation des offenen Leserahmens für das Polypeptid K8.1 (ORF K8.1). Der ORF K8.1 liegt zwischen dem ORF K8 und dem ORF52. Zusätzlich sind die Nucleotidpositionen der drei Leserahmen angegeben.
Figur 4 zeigt den Nachweis der Glykosilierung des immunogenen KSHV-Proteins von 35 bis 37 kDa (gp 35/37). Zur Auftrennung wurde ein 12 %iges Polyacrylamidgel verwendet. Im Westernblot wurde ein Serum von einem HIV-positiven, KS-positiven Patienten in einer Verdünnung von 1/200 verwendet. Es wurden je Spur jeweils 45 µg gesamtzelluläres Protein in SDS-Probenpuffer aufgetragen. Bei den einzelnen Spuren wurde aufgetragen:
M: Rainbow-Marker; Spur 1: HSB-2 Zellen (HHV-8 negativ, Kontrolle); Spur 2: nicht-induzierte BCBL-1 Zellen; Spur 3: BCBL-1 Zellen, 4 Tage induziert mit TPA, 20 ng/ml; Spur 4: BCBL-1 Zellen, 4 Tage induziert mit TPA nach Verdau mit PNGase F; Spur 5: "mock"-Verdau, d.h. identische Behandlung von BCBL-1 Zellen wie bei der in Spur 4 aufgetragenen Probe, doch ohne Zugabe von PNGase F.

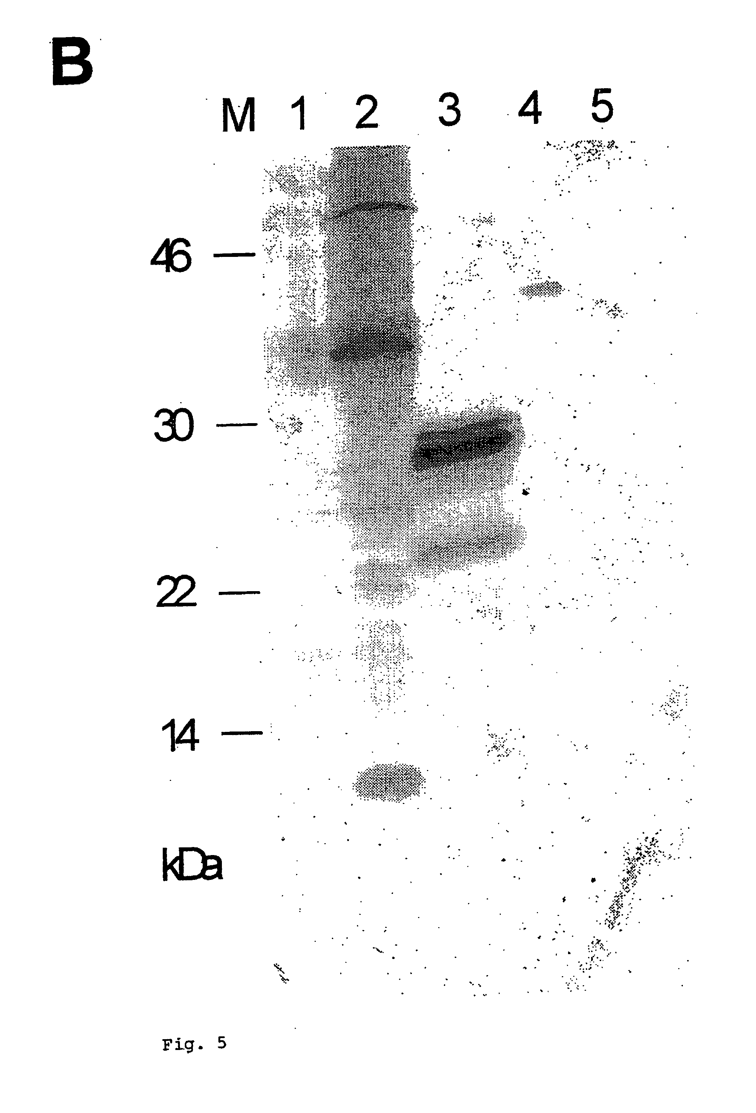

Figur 5 zeigt, daß rekombinantes K8.1 Polypeptid von einem Serum eines Kaposi-Sarkom-Patienten erkannt wird, das auch mit gp35/37 reagiert. Das Laufverhalten des in E.coli exprimierten Proteins gleicht dem des mit gp35/37 aus BCBL-1 Zellen nach Endoglycosidase Behandlung beobachteten.

Die Auftrennung erfolgte mit einem 15 %igen Polyacrylamidgel. Auf die einzelnen Spuren wurde aufgetragen:

M: Größenstandard; Spur 1: BJAB-Zellen; Spur 2: BCBL-1 Zellen, 4 Tage mit TPA induziert; Spur 3: rekombinantes Polypeptid K8.1, 200 ng; Spur 4: rekombinantes Fusionsprotein mit GST-Anteil, wobei der antigene Anteil von ORF65 stammt, 200 ng; Spur 5: rekombinantes vIL-6, 200 ng.

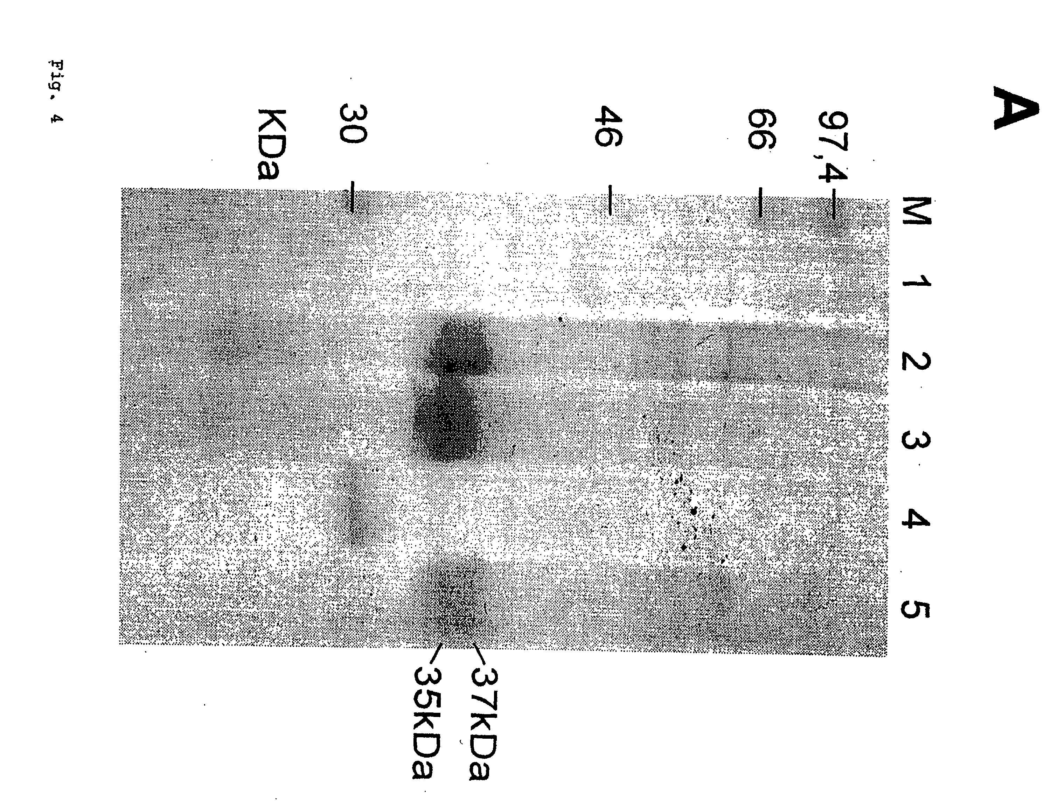

In Figur 5 wurde ein anderes Serum als in Figur 4 verwendet, bei dem - im Unterschied zu dem bei Figur 4 verwendeten - eine dritte Bande im Bereich von 35 bis 37 kDa sichtbar wird. Diese deutliche, scharfe Bande ist bei ca. 50 % aller Seren gut erkennbar. Sehr wahrscheinlich ist dies ein anderes virales Protein, denn im Unterschied zu gp35/37 handelt es sich um eine scharfe, für Glykoproteine untypische Bande. Darüber hinaus gibt es ein Serum von einem anderen HIV-positiven, Kaposi-Sarkom-Patienten, das beim Westernblot mit induzierten BCBL-1 Zellen nur diese relativ scharfe Bande von 36 kDa zeigt und nicht die beiden Banden von gp35/37. Dieses Serum erkennt das rekombinante Polypeptid K8.1 nicht.

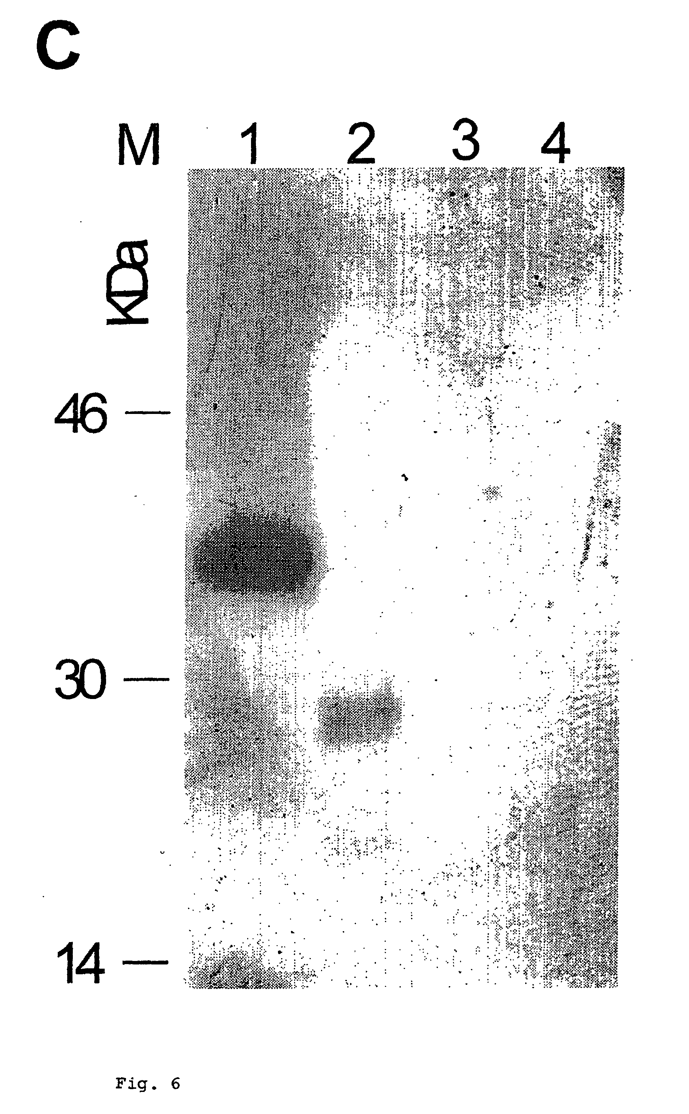

Figur 6 zeigt die Identität des erfindungsgemäßen Polypeptids K8.1 mit dem Glykoprotein gp35/37. Die Auftrennung erfolgte mit einem 15 %igen Polyacrylamidgel. Es wurden spezifische Antikörper gegen rekombinantes Polypeptid K8.1 aus dem Serum eines HIV-positiven Kaposi-Sarkom-Patienten durch Bindung an das rekombinante Polypeptid gereinigt. Diese wurden eingesetzt in einer Verdünnung von 1/200. Auf Spur 1 wurden BCBL-1 Zellen, 4 Tage mit TPA induziert, aufgetragen. Auf Spur 2 wurde rekombinantes Polypeptid K8.1 aufgetragen (200 ng). Auf Spur 3 wurde rekombinantes p18-GST aufgetragen in einer Menge von 200 ng. Bei p18-GST handelt es sich um ein Fusionsprotein mit einem GST-Anteil und einem Anteil von ORF65. Auf Spur 4 wurde rekombinantes vIL-6, 200 ng aufgetragen.

Die Erfindung wird durch die nachfolgenden Beispiele näher erläutert.

### Beispiel 1

### Anzucht der Zellen

Die KSHV (HHV-8) positiven BCBL-1 Zellen wurden über das ..AIDS Research and Reference Reagent"-Programm bezogen. Die HSB-1 und BJAB-Zellen sind von der ATCC ohne weiteres erhältlich. Die Zellen wurden in RPMI 1640-Medium mit 15 % fötalem Kälberserum, Glutamin und Gentamycin bei Standardbedingungen inkubiert. Zur TPA-Induktion wurden die Zellen bei ca. 1 x 10⁶ Zellen/ml mit 20 ng/TPA induziert. Für die Westernblots wurden die Zellen zweimal in PBS-Standardpuffer gewaschen, in 1/100 des Ausgangsvolumens PBS resuspendiert und die Proteinkonzentration mit dem "Pierce-Assay" nach Anweisung des Herstellers bestimmt.

### Beispiel 2

### Endoglycosidase F Verdau

BCBL-1 Zellen wurden für 4 Tage mit TPA induziert. Nach zweimaligem Waschen in PBS wurden die Zellen in 1 % des Ausgangsvolumens PBS resuspendiert. 45 µl dieser Zellsuspension, entsprechend 400 µg Gesamtprotein, wurden zunächst in einem 100 µl Ansatz mit Denaturierungspuffer (0,5 % SDS, 1 % β-Mercaptoethanol) 10 Minuten bei 96°C denaturiert. Nach Zugabe von 15 µl 10 % NP-40 (Biolabs) und 15 µl G7 Spaltpuffer (Biolabs) erfolgte der Verdau mit 10000 Einheiten PNGase F (New England Biolabs) für 1 Stunde bei 37°C. 17 µl dieses Reaktionsansatzes, entsprechend 45 µg zellulärem Protein, wurden für den Westernblot der Figur 4, Spur 4, verwendet.

### Beispiel 3

### Proteingele und Westernblot

12 und 15 %ige Proteingele mit einem Mischungsverhältnis Acrylamid/Bisacrylamid von 29/1 wurden nach Standardprotokollen hergestellt (Ausubel et al., Current Protocols in Molecular Biology, Wiley & Sons). Nach elektrophoretischer Auftrennung wurden die Proteine aus dem Polyacrylamidgel elektrophoretisch auf Nitrozellulose Membranen (Schleicher & Schüll) transferiert unter Verwendung einer Semi-Dry Blotkammer von Hoefer nach Anleitung des Herstellers. Vor der Inkubation mit dem humanen Serum wurden die Nitrozellulose Membranen zunächst über Nacht bei 4°C in ..Block Puffer" (150 mM NaCl, 20 mM Tris pH 7,5, 0,5 % Tween-20, 5 % Magermilchpulver) präinkubiert. Die Inkubation mit den humanen Seren erfolgte bei Raumtemperatur für 2 Stunden ebenfalls in Block Puffer, die Seren wurden stets 1/200 verdünnt. Nach 3maligem Waschen in TBS-Tween (150 mM NaCl, 20 mM Tris pH 7,5, 0,5 % Tween) für jeweils 15 Minuten wurden die Seren mit dem 2. Antikörper für 60 Minuten inkubiert (Anti-Human alkalische Phosphatase konjugierte Antikörper vom Kaninchen, Dako). Die Membranen wurden dann zunächst 3x TBS-Tween gewaschen, dann 1x in TBS (150 mM NaCl, 20 mM Tris pH 7,5) gewaschen und mit einer Substrat-Lösung (RAD-Free BCIP/NBT Tabletten, Schleicher & Schüll) nach den Angaben des Herstellers gefärbt.

### Beispiel 4

### Klonierung und prokaryonte Expression von KSHV Polypeptid K8.1

Mit den synthetischen Oligonukleotiden K8-ImBam (gtgcggatccaattgtcccacgtatcgttc) und K8-lrHind (ggcaaagcttggcacacggttactagcacc) wurde ein Fragment des offenen Leserahmens K8.1 amplifiziert, das den kodierenden Bereich ab Aminosäure 27 enthält. Dies entspricht dem vermutlichen kompletten Protein nach Abspaltung des Signalpeptids. Für die PCR wurde in einem 100 µl Ansatz 100 ng DNA aus BCBL-1 Zellen mit je 100 ng der obigen Primer amplifiziert in 30 Zyklen (95°C 20 sec, 50°C 20 sec, 72°C 80 sec). Das erhaltene DNA-Fragment wurde mit HindIII und BamHI verdaut. 50 ng des gespaltenen Amplifikats wurden mit 100 ng des ebenfalls BamHI und HindIII gespaltenen Vektors pQE9 (Quiagen) nach den Anweisungen des Herstellers ligiert (T4 DNA Ligase, Gibco BRL). Nach Transfektion des Ligationsansatzes in den E. coli Stamm JM 109 (Stratagene Inc., La Jolla) wurde ein positiver Klon (pQK8-Im) selektiert und die Nucleinsäuresequenz des viralen Anteils sowie der für die prokaryonte Expression wichtigen Vektorabschnitte (Promotor, Histidin-Taq) bestimmt. Die Nucleinsäuresequenz wurde mit dem ABI377 Sequenzierautomaten (ABI Inc., Foster City, USA) nach Protokollen des Herstellers überprüft. Zur Herstellung und Aufreinigung des rekombinanten Proteins K8.1 in E.coli JM 109 wurden die Bakterien mit 2 mM IPTG induziert, lysiert und unter denaturierenden Bedingungen über Metall-Affinitätschromatographie aufgereinigt wie vom Hersteller (Quiagen AG, Hilden: ..The Quiaexpressionist", Ausgabe Sommer 1992, S. 45 ff.) beschrieben. Die Bakterienkultur wurde bei einer OD600 = 0,6 mit 2 mM IPTG induziert, nach 3 h Inkubation bei 37°C abzentrifugiert und in 6 M Guanidiniumrhodanid, 0,1 M Natriumphosphat, 10 mM Tris pH 8,0 lysiert. Der geklärte Überstand wurde an Ni-NTA Matrix absorbiert und mit steigenden Konzentrationen von Imidazol in 8 M Harnstoff, pH 6,5, eluiert. Die Fraktionen wurden im Polyacrylamidgel (15 %) nach Comassie-Färbung auf Proteingehalt untersucht. Der überwiegende Teil des rekombinanten K8.1 eluierte zwischen 200 und 400 mM Imidazol. Für die weitere Verwendung in Westernblot und ELISA wurden die Protein enthaltenden Fraktionen gepoolt und über Nacht gegen 10 mM Tris pH 7,5 dialysiert. Das rechnerische Molekulargewicht des so exprimierten Proteins beträgt 20,4 kDa. Das scheinbare Molekulargewicht im Polyacrylamidgel liegt bei etwa 28 kDa.

### Beispiel 5

### Absorption von Polypeptid K8.1 spezifischen Antikörpern aus humanem Serum

400 µg prokaryont exprimiertes (Plasmid pQK8-1m) und affinitätschromatographisch gereinigtes K8.1 Polypeptid wurde in einem 15 % Polyacrylamidgel aufgetrennt und wie beschrieben auf Nitrocellulose Membran transferiert. Durch Färbung mit Ponceau-Rot wurde die Laufhöhe des prokaryont exprimierten Proteins bestimmt und der entsprechende Bereich des Filters ausgeschnitten. Nach Entfernen der Ponceau-Rot-Färbung durch mehrfaches Waschen in 150 mM NaCl, 20 mM Tris, 1 % Tween wurde der NC-Streifen zunächst 4 h bei 20°C in Block-Puffer inkubiert. Darauf folgend wurde der NC-Streifen mit dem immobilisierten K8.1 Protein 16 Stunden bei 4°C mit dem 1/200 verdünnten Serum eines Kaposi-Sarkom-Patienten inkubiert. Nach 4-maligem Waschen mit TBS-Tween wurden die gebundenen Antikörper mit 100 mM Glycin, pH 2,9 eluiert. Dabei wurde der NC-Streifen nur 1 Minute mit der Glycin-Lösung inkubiert und die Lösung sofort mit 1/10 Volumen 1 M Tris pH 9 neutralisiert. Die so aufgereinigten monospezifischen Antikörper wurden zum Nachweis der Identität von K8.1 mit gp35/37 in dem in Abbildung C gezeigten Westernblot eingesetzt (Verdünnung 1/200 bezogen auf das Volumen des absorbierten Serums).

### Beispiel 6

### Ergebnisse der Westernblot-Tests

Bei den Versuchen wurde die immunologische Reaktivität der verschiedenen Antigene mit Seren verschiedener Spender untersucht. Die Ergebnisse der Westernblots sind in der Tabelle 1 zusammengefaßt.

**Tabelle 1:**

| Reaktivität im Westernblot | | | | |
|---|---|---|---|---|
| Serum | Kategorie/Status | gp35/37nat | gp35/37rec | GST-ORF65 |
| 1 | HIV+, KS+ | + | + | + |
| 2 | HIV+, KS+ | + | + | + |
| 3 | HIV+, KS+ | + | + | + |
| 4 | HIV+, KS+ | + | + | + |
| 5 | HIV+, KS+ | + | + | + |
| 6 | HIV+, KS+ | + | + | + |
| 7 | HIV+, KS+ | + | + | ? |
| 8 | HIV+, KS+ | + | + | ? |
| 9 | HIV+, KS+ | + | + | - |
| 10 | HIV+, KS+ | + | + | - |
| 11 | HIV+, KS+ | - + | + | - |
| 12 | HIV+, KS+ | + | + | - |
| 13 | HIV+, KS+ | + | + | - |
| 14 | HIV+, KS+ | + | + | - |
| 15 | HIV+, KS+ | + | + | - |
| 16 | HIV+, KS+ | + | + | - |
| 17 | HIV+, KS+ | + | + | - |
| 18 | HIV+, KS+ | + | + | - |
| 19 | HIV+, KS+ | + | + | - |
| 20 | HIV+, KS+ | + | + | - |
| 21 | HIV+, KS+ | + | + | - |
| 22 | HIV+, KS+ | ? | + | ? |
| 23 | HIV+, KS+ | - | - | ? |
| 24 | HIV+, KS+. | - | - | - |
| 25 | HIV+, KS+ | - | - | - |
| 26 | HIV+, KS+ | - | - | - |
| 27 | HIV+, KS+ | - | - | - |
| 28 | HIV -, KS+ | + | + | - |
| 29 | HIV -, KS+ | + | + | - |
| 30 | HIV+, KS - | - | - | - |
| 31 | HIV+, KS - | - | - | - |
| 32 | HIV+, KS - | - | - | - |
| 33 | HIV+, KS - | - | - | - |
| 34 | HIV+, KS - | - | - | - |
| 35 | HIV+, KS - | - | - | - |
| 36 | HIV+, KS - | - | - | - |
| 37 | HIV+, KS - | + | ? | + |
| 38 | HIV -, KS - | - | - | - |
| 39 | HIV -, KS - | - | - | - |
| 40 | HIV -, KS - | - | - | ? |
| 41 | EBV-Primärinf. | - | - | + |

Die Tabelle 1 zeigt die Westernblot-Ergebnisse mit den HHV-8-Antigenen und 41 Humanseren verschiedener Kategorie. Reaktionen: + = reaktive Bande, d.h. positive Reaktion, ? = fragliche Reaktion,, - = keine Bande, d.h. negative Reaktion. Als Antigene eingesetzt wurden: gp35/37 nat = natürliches gp35/37 in Extrakten aus induzierten BCBL-1 Zellen, ungereinigt; gp35/37 rec = rekombinantes Histidin-Tag-Protein, ohne Leader-Sequenz, gereinigt; GST-ORF65 = rekombinantes GST (Glutathion S-Transferase)-Fusionsprotein mit Polypeptid aus dem ORF65 des KSHV, gereinigt.

Die Ergebnisse lassen folgende Schlußfolgerungen zu:

Das natürliche und das rekombinante gp35/37 Protein zeigten vollkommene Übereinstimmungen mit den verschiedenen Seren, sieht man von zwei fraglichen Reaktionen (Nr. 22 und 37) einmal ab. Dies ist ein weiteres Indiz für die Identität. Das gp35/37 reagierte mit 24 von 29 Seren von Kaposi-Patienten positiv, während ORF65 nur maximal 10/29 erfaßte. Dies belegt eindeutig die Überlegenheit der erfindungsgemäßen Polypeptide. Nur eines von 12 KS-negativen Seren (Nr. 37) hatte mit gp35/37 reagiert. Dieses Serum eines HIV-Positiven reagierte auch mit GST-ORF65. Da HHV-8 Antikörper schon vor einer klinischen Manifestation nachweisbar werden, sind solche Ergebnisse zu erwarten.

ORF65 reagierte zusätzlich mit dem Serum eines Patienten mit Infektiöser Mononuklease = EBV-Primärinfektion (Nr. 41). Zwar ist eine positive Reaktion hervorgerufen durch den Fusionspartner nicht ausgeschlossen, jedoch war das gp35/37 in diesen Experimenten das eindeutig bessere Antigen.

### Beispiel 7

### Reaktivität des gereinigten rekombinanten gp35/37 im ELISA

Je 100 ng der gereinigten rekombinanten Antigene gp35/37, EBV GST p18 und GST ORF65 gelöst in 100 M 0,01 M Carbonatpuffer pH 9,5 wurden jeweils in die Vertiefung von Mikrotiterplatten (Nunc, Maxisorp) gegeben und 16 h in einer geerdeten, feuchten Kammer inkubiert. Nach Zugabe von 100 µl einer Kalbsserumhaltigen Nachbeschichtungslösung, wurde die Inkubation für 2 weitere Stunden fortgesetzt. Danach wurden die Platten entleert und sorgfältig ausgeschlagen. Anschließend wurden die beschichteten Platten für die eigentliche Testung verwendet, oder nach Trocknung im Vakuumschrank, mit anschließendem Einschweißen in einem Folienschlauch bis zum späteren Gebrauch bei -20°C gelagert. Für die Testdurchführung wurden die Testnäpfchen der ELISA-Platten mit 100 µl einer 1:21 verdünnten Serumlösung gefüllt, mit einer Plastikfolie abgeklebt und bei 37°C im Brutschrank (Binder, BED53) für 1 h inkubiert. Nach dreimaligem Waschen im Biotest Washer II erfolgte die Konjugatinkubation mit 100 µl von gegen humanes IgG gerichteten, mit Peroxidase-markierten, monoklonalen Antikörpern aus der Maus (30 min bei 37°). Nach abermaligem Waschen wurden die gebundenen Antikörper durch die Enzymaktivität der Peroxidase mittels 100 µl 3,3',5,5'-Tetramethylbenzidine (TMB, Sigma) sichtbar gemacht. Die Chromogenreaktion dauerte 30 min bei Raumtemperatur und wurde durch die Zugabe von 100 µl 1 N Schwefelsäure beendet. Die optische Dichte (OD) der einzelnen Proben wurde bei 495 nm (Referenz: 620 nm) im Anthos HTII ELISA-Reader ermittelt. Alle OD-Werte größer als 400 mOD wurden im Fall der HHV8 spezifischen Tests (gp35/37 und ORF65) als positiv bewertet. Für den EBV abhängigen Test wurde ein "cut off" von 0,15 OD definiert.

### Ergebnisse

Um die antigenen Eigenschaften des rekombinanten gp35/37, die in Westernblot-Experimenten ermittelt worden waren, zu bestätigen, wurden ELISA Tests durchgeführt. Dazu wurden Seren von HIV-Patienten mit Kaposi-Sarkom (KS+/HIV+) im Vergleich mit KS-/HIV+ und mit Seren von gesunden Blutspendern (BS) getestet. Alle Werte, die ein Verhältnis von OD zu "cut off" (S/C-Ratio) von größer als 1 aufwiesen, wurden als positiv gewertet. Von den KS+/HIV+ Proben waren mit gp35/37 insgesamt 9 von 10 Seren (90 %) positiv, mit dem rekombinanten ORF 65 Antigen 8/10 (80%) positiv. Damit ergab sich mit gp35/37 gegenüber ORF 65 ein Sensitivitätsvorteil von 10 %. Von den KS-/HIV+ Proben war mit beiden Antigenen jeweils eine Probe (Serum LD) reaktiv. Möglicherweise handelt es sich dabei um eine Blutprobe eines HIV-Patienten, der zu einem späteren Zeitpunkt ein Kaposi-Karzinom entwickelt hat. Das Testen einer Folgeprobe war zum Zeitpunkt der Erfindung nicht möglich. Um die Spezifität beider Antigene zu vergleichen, wurden Blutproben von gesunden Individuen untersucht. In diesem Kollektiv sollten erwartungsgemäß keine Antikörper gegen HHV8 nachweisbar sein. Von 12 getesteten Seren von Blutspendern (BS) waren mit dem gp35/37 erwartungsgemäß alle Proben negativ. Mit dem ORF65-Test wurde ein Serum (Suhl 792) positiv bewertet, ein Umstand, der auf eine größere Unspezifität des Antigens ORF65 hinweist. Generell zeigten die Ergebnisse, dass unter den verwendeten Bedingungen die Trennung zwischen positiven und negativen OD-Werten mit gp35/37 größer war als mit ORF65. Die S/C-Ratios sind mit gp35/37 deutlich höher, worin sich die relativ zu ORF65 bessere immunogene Reaktivität des Antigens ausdrückt.

Zahlreiche Leserahmen von HHV8 weisen Sequenzhomologien zum EBV-Genom auf. Dazu sollte überprüft werden, ob Seren mit hohem Antikörpertiter gegen EBV mit gp35/37 kreuzreagieren. Hierzu wurden 7 Seren von Nasopharynx Karzinom (NPC) Patienten, die bekannterweise hohe Titer gegen EBV Antigene aufweisen, getestet. Als positiv Kontrolle wurde das rekombinante EBV Virus Kapsid Protein (VCA) p18 als GST-Fusionsprotein verwendet. Alle NPC-Seren waren negativ mit gp35/37. Drei Seren C6, C19 und C28 waren positiv mit ORF65 und alle Seren waren hoch positiv mit EBV p18 VCA-Antigen. Die Ergebnisse zeigten, daß eine mögliche Kreuzraktivität mit gp35/37 ausgeschlossen werden kann. Hingegen konnte bei dem HHV 8 Antigen ORF65 eine Kreuzreaktivität beobachtet werden.

Zusammenfassend ergibt sich, daß das rekombinante Antigen gp35/37 sowohl eine bessere Reaktivität als auch eine höhere Spezifität gegenüber ORF65 vermittelt. Um die Reaktivitätsvorteile des rekombinanten gp35/37 zu untermauern, wurden 50 kommerziell erhältliche Seren (BioClinical Partners) von HIV-positiven homosexuellen Männern (KS-Risiko) aus San Francisco getestet. Nach Angaben in der Literatur ist dieses Patientenkollektiv mit einem erhöhten Risiko für die Ausbildung eines Kaposi-Sarkoms behaftet. Von den 50 getesteten Seren waren mit gp35/37 29 (58 %) positiv, mit ORF65 insgesamt 7 (14 %). Die Ergebnisse zeigen, daß das Antigen gp35/37 als diagnostischer Marker eine hohe Sensitivität vermittelt und als zuverlässiger Seromarker eingesetzt werden kann.

Die Ergebnisse dieses Versuchs wurden in den Tabellen 2 und 3 zusammengefaßt.

**Tabelle 2**

| | | EBV | HHV 8 | | | EBV | HHV 8 |
|---|---|---|---|---|---|---|---|
| | gp35/37 | GST p18 | GST ORF65 | | gp35/37 | GST p18 | GST ORF 65 |
| | | | | | | | |
| KS+/HIV+ | | | | BS | | | |
| 26295 | 6,9 | 20,0 | 1,3 | Suhl 789 | 0,2 | 20,0 | 0,6 |
| 22675 | 7,0 | 20,0 | 1,2 | Suhl 790 | 0,3 | 12,9 | 0,4 |
| 26673 | 7,5 | 20,0 | 1,2 | Suhl 791 | 0,3 | 20,0 | 0,7 |
| 43742 | 7,5 | 20,0 | 2,0 | Suhl 792 | 0,2 | 20,0 | 1,0 |
| 23971 | 6,9 | 20,0 | 0,5 | Suhl 793 | 0,1 | 18,4 | 0,3 |
| 43630 | 4,5 | 20,0 | 3,0 | Suhl 794 | 0,2 | 20,0 | 0,4 |
| 5942 | 0,5 | 20,0 | 0,5 | Suhl 795 | 0,2 | 20,0 | 0,3 |
| SH | 3,0 | 20,0 | 1,1 | Suhl 796 | 0,2 | 20,0 | 0,4 |
| BP | 1,1 | 20,0 | 1,2 | Suhl 797 | 0,1 | 13,9 | 0,4 |
| AC(53) | 6,5 | 20,0 | 1,8 | Suhl 798 | 0,2 | 18,0 | 0,4 |
| | | | | Suhl 799 | 0,2 | 20,0 | 0,4 |
| KS-/HIV+ | | | | Suhl 800 | 0,2 | 20,0 | 0,4 |
| MM | 0,4 | 20,0 | 0,3 | NPC | | | |
| WU | 0,6 | 20,0 | 0,8 | C 6 | 0,4 | 17,6 | 1,7 |
| FP | 0,6 | 13,1 | 0,6 | C 9 | 0,2 | 18,6 | 0,8 |
| L D | 1,0 | 20,0 | 3,4 | C 10 | 0,3 | 20,0 | 0,8 |
| RK | 0,7 | 0,9 | 0,6 | C 19 | 0,3 | 20,0 | 1,1 |
| D J | 0,5 | 1,0 | 0,6 | C 21 | 0,2 | 20,0 | 0,6 |
| A E | 0,6 | 20,0 | 0,4 | C 22 | 0,4 | 20,0 | 0,7 |
| AP | 0,5 | 4,2 | 0,5 | C 28 | 0,2 | 20,0 | 1,1 |

**Tabelle 3**

| | | EBV | HHV8 | | | EBV | HHV8 |
|---|---|---|---|---|---|---|---|
| | gp35/37 | GST p18 | GST ORF 65 | | gp35/37 | GST p18 | GST ORF 65 |
| KS-Risiko | | | | | | | |
| 0744 | 0,8 | 18,7 | 0,5 | 0773 | 6,1 | 20,0 | 0,6 |
| 0745 | 7,5 | 17,3 | 1,2 | 0774 | 4,0 | 19,0 | 0,3 |
| 0746 | 0,7 | 20,0 | 0,3 | 0775 | 7,5 | 18,4 | 0,3 |
| 0747 | 0,5 | 17,3 | 0,2 | 0776 | 0,8 | 18,7 | 0,4 |
| 0748 | 0,6 | 20,0 | 0,5 | 0777 | 7,5 | 18,5 | 0,6 |
| 0749 | 7,5 | 18,3 | 0,4 | 0778 | 0,5 | 17,9 | 0,3 |
| 0750 | 2,7 | 15,2 | 1,8 | 0779 | 7,5 | 18,4 | 1,7 |
| 0751 | 0,7 | 18,5 | 0,3 | 0780 | 7,5 | 18,6 | 0,7 |
| 0752 | 0,7 | 20,0 | 0,3 | 0781 | 1,1 | 17,6 | 0,5 |
| 0753 | 2,1 | 20,0 | 0,4 | 0782 | 2,6 | 20,0 | 0,4 |
| 0754 | 2,0 | 20,0 | 0,7 | 0783 | 7,5 | 18,4 | 1,5 |
| 0755 | 0,6 | 16,4 | 0,3 | 0784 | 1,0 | 20,0 | 0,3 |
| 0756 | 0,5 | 20,0 | 0,3 | 0785 | 7,5 | 20,0 | 0,8 |
| 0757 | 2,6 | 20,0 | 0,3 | 0786 | 7,5 | 20,0 | 0,7 |
| 0758 | 0,7 | 18,4 | 0,3 | 0787 | 6,1 | 20,0 | 0,3 |
| 0759 | 0,5 | 18,5 | 0,4 | 0788 | 0,8 | 20,0 | 0,2 |
| 0760 | 1,3 | 20,0 | 0,4 | 0789 | 2,1 | 16,7 | 0,3 |
| 0761 | 7,5 | 20,0 | 0,9 | 0790 | 0,3 | 19,0 | 0,5 |
| 0762 | 7,5 | 20,0 | 1,2 | 0791 | 0,6 | 20,0 | 0,5 |
| 0763 | 0,8 | 20,0 | 1,1 | 0792 | 0,9 | 18,6 | 0,7 |
| 0764 | 2,5 | 20,0 | 0,3 | 0793 | 6,4 | 17,8 | 1,0 |
| 0765 | 0,9 | 20,0 | 0,3 | | | | |
| 0766 | 7,5 | 20,0 | 0,7 | % | 58% | 100% | 14% |
| 0767 | 1,7 | 20,0 | 0,2 | | | | |
| 0768 | 0,9 | 20,0 | 0,5 | | | | |
| 0769 | 0,5 | 20,0 | 0,2 | | | | |
| 0770 | 0,3 | 20,0 | 0,2 | | | | |
| 0771 | 3,2 | 20,0 | 0,3 | | | | |
| 0772 | 2,0 | 20,0 | 0,3 | | | | |

## Patentansprüche

1. Polypeptid, **dadurch gekennzeichnet, daß** es eine Teilsequenz von wenigstens 10 aufeinanderfolgenden Aminosäuren der Sequenz aufweist.

2. Polypeptid, **dadurch gekennzeichnet, daß** es eine Teilsequenz von wenigstens 10 aufeinanderfolgenden Aminosäuren der Sequenz aufweist.

3. Polypeptid nach Anspruch 1 oder 2, **dadurch gekennzeichnet, daß** es eine Teilsequenz von wenigstens 14 aufeinanderfolgenden Aminosäuren aufweist.

4. Polypeptid nach Anspruch 1 oder 2, **dadurch gekennzeichnet, daß** es eine Teilsequenz von wenigstens 25 aufeinanderfolgenden Aminosäuren aufweist.

5. Polypeptid nach Anspruch 1 oder 2, **dadurch gekennzeichnet, daß** es eine Teilsequenz von wenigstens 30 aufeinanderfolgenden Aminosäuren aufweist.

6. Polypeptid nach Anspruch oder 2, **dadurch gekennzeichnet, daß** es eine Teilsequenz von wenigstens 40 aufeinanderfolgenden Aminosäuren aufweist.

7. Polypeptid nach einem der vorhergehenden Ansprüche, **dadurch gekennzeichnet, daß** das Polypeptid von dem Kaposi-Sarkom assoziierten Herpes-Virus kodiert wird.

8. Polypeptid nach einem der vorhergehenden Ansprüche, **dadurch gekennzeichnet, daß** es mit solchen Antikörpern reagiert, die spezifisch für das Kaposi-Sarkom assoziierte Herpes-Virus sind.

9. Polypeptid nach einem der Ansprüche 1 bis 8, **dadurch gekennzeichnet, daß** es chemisch synthetisiert wurde.

10. Polypeptid nach einem der Ansprüche 1 bis 8, **dadurch gekennzeichnet, daß** es gentechnisch in einer prokaryontischen Wirtszelle exprimiert wurde.

11. Polypeptid nach einem der Ansprüche 1 bis 8, **dadurch gekennzeichnet, daß** es gentechnisch in einer eukaryontischen Wirtszelle exprimiert wurde.

12. Testkit zum Nachweis einer Infektion mit einem Kaposi-Sarkom assoziierten Herpes-Virus in einem Menschen oder Säugetier, **dadurch gekennzeichnet, daß** es ein Polypeptid nach einem der Ansprüche 1 bis 11 umfaßt.

13. Testkit nach Anspruch 12, **dadurch gekennzeichnet, daß** es eine weitere Komponente zum Nachweis des Komplexes umfassend ein Polypeptid nach einem der Ansprüche 1 bis 11 und wenigstens eines hieran spezifisch gebundenen Antikörpers aufweist.

14. Testkit nach Anspruch 13, **dadurch gekennzeichnet, daß** die Komponente zum Nachweis des Komplexes ein Anti-Antikörper ist.

15. Testkit nach Anspruch 13, **dadurch gekennzeichnet, daß** die Komponente zum Nachweis des Komplexes ein weiterer Antikörper gegen ein Polypeptid nach einem der Ansprüche 1 bis 11 ist.

16. Testkit nach einem der Ansprüche 13 bis 15, **dadurch gekennzeichnet, daß** die Komponente zum Nachweis des Komplexes markiert ist.

17. Testkit nach Anspruch 16, **dadurch gekennzeichnet, daß** die Komponente zum Nachweis des Komplexes mit einem Enzym markiert ist.

18. Testkit nach Anspruch 12, **dadurch gekennzeichnet, daß** das Polypeptid markiert ist.

19. Verwendung eines Polypeptids nach einem der Ansprüche 1 bis 11 zum Nachweis einer Infektion mit einem Kaposi-Sarkom assoziierten Herpes-Virus.

20. Nucleinsäurekonstrukt zur gentechnologischen Herstellung eines Polypeptids nach einem der Ansprüche 1 bis 11, **dadurch gekennzeichnet, daß** es die Nucleinsäuresequenz oder eine Teilsequenz davon mit wenigstens 30 Nucleotiden umfaßt.

21. Nucleinsäurekonstrukt nach Anspruch 20, **dadurch gekennzeichnet, daß** es ein Expressionsvektor ist.

22. Verwendung einer Partialsequenz mit 15 bis 35 Nucleotiden aus der Nucleinsäuresequenz oder der hierzu komplementären Nucleinsäuresequenz zum Nachweis einer Infektion mit einem Kaposi-Sarkom assoziierten Herpes-Virus mit Hilfe der Polymerase Kettenreaktion.

23. Impfstoff, **dadurch gekennzeichnet, daß** er ein Polypeptid nach einem der Ansprüche 1 bis 11 umfaßt.

24. Antikörper, **dadurch gekennzeichnet, daß** er gegen ein Polypeptid nach einem der Ansprüche 1 bis 11 gerichtet ist.

25. Antikörper nach Anspruch 24, **dadurch gekennzeichnet, daß** es ein monoklonaler Antikörper ist.

26. Antikörper nach Anspruch 25, **dadurch gekennzeichnet, daß** es ein humanisierter monoklonaler Antikörper ist.

## Claims

1. Polypeptide, **characterized in that** it has a part-sequence of at least 10 consecutive amino acids from the sequence

2. Polypeptide, **characterized in that** it has a part-sequence of at least 10 consecutive amino acids of the sequence

3. Polypeptide according to claim 1 or 2, **characterized in that** it has a part-sequence of at least 14 consecutive amino acids.

4. Polypeptide according to claim 1 or 2, **characterized in that** it has a part-sequence of at least 25 consecutive amino acids.

5. Polypeptide according to claim 1 or 2, **characterized in that** it has a part-sequence of at least 30 consecutive amino acids.

6. Polypeptide according to claim 1 or 2, **characterized in that** it has a part-sequence of at least 40 consecutive amino acids.

7. Polypeptide according to any of the preceding claims, **characterized in that** the polypeptide is encoded by the Kaposi sarcoma-associated herpes virus.

8. Polypeptide according to any of the preceding claims, **characterized in that** it reacts with those antibodies which are specific for the Kaposi sarcoma-associated herpes virus.

9. Polypeptide according to any of claims 1 to 8, **characterized in that** it has been synthesized chemically.

10. Polypeptide according to any of claims 1 to 8, **characterized in that** it has been expressed by genetic manipulation in a prokaryotic host cell.

11. Polypeptide according to any of claims 1 to 8, **characterized in that** it has been expressed by genetic manipulation in a eukaryotic host cell.

12. Test kit for detecting an infection with a Kaposi sarcoma-associated herpes virus in a human or mammal, **characterized in that** it comprises a polypeptide according to any of claims 1 to 11.

13. Test kit according to claim 12, **characterized in that** it has another component for detecting the complex comprising a polypeptide according to any of claims 1 to 11 and at least one antibody specifically bound thereto.

14. Test kit according to claim 13, **characterized in that** the component for detecting the complex is an anti-antibody.

15. Test kit according to claim 13, **characterized in that** the component for detecting the complex is another antibody against a polypeptide according to any of claims 1 to 11.

16. Test kit according to any of claims 13 to 15, **characterized in that** the component for detecting the complex is labeled.

17. Test kit according to claim 16, **characterized in that** the component for detecting the complex is labeled with an enzyme.

18. Test kit according to claim 12, **characterized in that** the polypeptide is labeled.

19. Use of a polypeptide according to any of claims 1 to 11 for detecting an infection with a Kaposi sarcoma-associated herpes virus.

20. Nucleic acid construct for the preparation by genetic manipulation of a polypeptide according to any of claims 1 to 11, **characterized in that** it comprises the nucleic acid sequence or a part-sequence thereof with at least 30 nucleotides.

21. Nucleic acid construct according to claim 20, **characterized in that** it is an expression vector.

22. Use of a partial sequence with 15 to 35 nucleotides from the nucleic acid sequence or from the nucleic acid sequence complementary thereto for detecting an infection with a Kaposi sarcoma-associated herpes virus by means of the polymerase chain reaction.

23. Vaccine, **characterized in that** it comprises a polypeptide according to any of claims 1 to 11.

24. Antibody, **characterized in that** it is directed against a polypeptide according to any of claims 1 to 11.

25. Antibody according to claim 24, **characterized in that** it is a monoclonal antibody.

26. Antibody according to claim 25, **characterized in that** it is a humanized monoclonal antibody.

## Revendications

1. Polypeptide, **caractérisé en ce qu'**il présente une séquence partielle d'au moins 10 acides aminés successifs de la séquence:

2. Polypeptide, **caractérisé en ce qu'**il présente une séquence partielle d'au moins 10 acides aminés successifs de la séquence:

3. Polypeptide suivant l'une des revendications 1 et 2, **caractérisé en ce qu'**il présente une séquence partielle d'au moins 14 acides aminés successifs.

4. Polypeptide suivant l'une des revendications 1 et 2, **caractérisé en ce qu'**il présente une séquence partielle d'au moins 25 acides aminés successifs.

5. Polypeptide suivant l'une des revendications 1 et 2, **caractérisé en ce qu'**il présente une séquence partielle d'au moins 30 acides aminés successifs.

6. Polypeptide suivant l'une des revendications 1 et 2, **caractérisé en ce qu'**il présente une séquence partielle d'au moins 40 acides aminés successifs.

7. Polypeptide suivant l'une des revendications précédentes, **caractérisé en ce que** le polypeptide est codé par le virus d'herpès associé au sarcome de Kaposi.

8. Polypeptide suivant l'une des revendications précédentes, **caractérisé en ce qu'**il réagit avec les anticorps qui sont spécifiques du virus d'herpès associé au sarcome de Kaposi.

9. Polypeptide suivant l'une des revendications 1 à 8, **caractérisé en ce qu'**il a été synthétisé par voie chimique.

10. Polypeptide suivant l'une des revendications 1 à 8, **caractérisé en ce qu'**il a été exprimé par ingénierie génétique dans une cellule hôte procaryote.

11. Polypeptide suivant l'une des revendications 1 à 8, **caractérisé en ce qu'**il a été exprimé par ingénierie génétique dans une cellule hôte eucaryote.

12. Trousse destinée à déceler une infection par un virus d'herpès associé au sarcome de Kaposi chez un être humain ou un mammifère, **caractérisée en ce qu'**elle comporte un polypeptide suivant l'une des revendications 1 à 11.

13. Trousse suivant la revendication 12, **caractérisée en ce qu'**elle présente un autre composant destiné à déceler le complexe comportant un polypeptide suivant l'une des revendications 1 à 11 et au moins un anticorps fixé de manière spécifique sur lui.

14. Trousse suivant la revendication 13, **caractérisée en ce que** le composant destiné à déceler le complexe est un anti-anticorps.

15. Trousse suivant la revendication 13, **caractérisée en ce que** le composant destiné à déceler le complexe est un autre anticorps contre un polypeptide suivant l'une des revendications 1 à 11.

16. Trousse suivant l'une des revendications 13 à 15, **caractérisée en ce que** le composant destiné à déceler le complexe est marqué.

17. Trousse suivant la revendication 16, **caractérisée en ce que** le composant destiné à déceler le complexe est marqué par une enzyme.

18. Trousse suivant la revendication 12, **caractérisée en ce que** le polypeptide est marqué.

19. Utilisation d'un polypeptide suivant l'une des revendications 1 à 11, pour déceler une infection par un virus d'herpès associé au sarcome de Kaposi.

20. Structure d'acides nucléiques pour la préparation par ingénierie génétique d'un polypeptide suivant l'une des revendications 1 à 11, **caractérisée en ce qu'**elle comporte la séquence d'acides nucléiques : ou une séquence partielle de celle-ci comportant au moins 30 nucléotides.

21. Structure d'acides nucléiques suivant la revendication 20, **caractérisée en ce qu'**elle est un vecteur d'expression.

22. Utilisation d'une séquence partielle comportant 15 à 35 nucléotides de la séquence d'acides nucléiques : ou de la séquence d'acides nucléiques qui lui est complémentaire, pour déceler une infection par un virus d'herpès associé au sarcome de Kaposi, à l'aide de la réaction en chaîne de polymérase.

23. Vaccin, **caractérisé en ce qu'**il comporte un polypeptide suivant l'une des revendications 1 à 11.

24. Anticorps, **caractérisé en ce qu'**il est dirigé contre un polypeptide suivant l'une des revendications 1 à 11.

25. Anticorps suivant la revendication 24, **caractérisé en ce qu'**il est un anticorps monoclonal.

26. Anticorps suivant la revendication 25, **caractérisé en ce qu'**il est un anticorps monoclonal humanisé.


(1) ALLGEMEINE INFORMATION:
   ANMELDER:
      (A) NAME: Biotest AG
      (B) STRASSE: Landsteinerstr. 5
      (C) ORT: Dreieich
      (E) LAND: Deutschland
      (F) POSTLEITZAHL: 63303
   ANMELDETITEL:
      Von Kaposi-Sarkom assoziierten Herpes-
      Virus kodierte Polypeptide und deren
      Verwendung in Diagnostik und Therapie
   ANZAHL DER SEQUENZEN: 2
   COMPUTER-LESBARE FORM:
      (A) DATENTRÄGER: Floppy Disk
      (B) COMPUTER: IBM PC compatible
      (C) BETRIEBSSYSTEM: PC-DOS/MS-DOS
      (D) SOFTWARE: PADAT Sequenzmodul Version 1.0
(2) INFORMATION ZU SEQ ID NO: 1:
   (i) SEQUENZ CHARAKTERISTIKA:
      (A) LÄNGE: 594 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 1:
   ATGAGTTCCA CACAGATTCG CACAGAAATC CCTGTGGCGC TCCTAATCCT ATGCCTTTGT     60
   CTGGTGGCGT GCCATGCCAA TTGTCCCACG TATCGTTCGC ATTTGGGATT CTGGCAAGAG    120
   GGTTGGAGTG GACAGGTTTA TCAGGACTGG CTAGGCAGGA TGAACTGTTC CTACGAGAAT    180
   ATGACGGCCC TAGAGGCCGT CTCCCTAAAC GGGACCAGAC TAGCAGCTGG ATCTCCGTCG    240
   AGTGAGTATC CAAATGTCTC CGTATCTGTT GAAGATACGT CTGCCTCTGG GTCTGGAGAA    300
   GATGCAATAG ATGAATCGGG GTCGGGGGAG GAAGAGCGTC CCGTGACCTC CCACGTGACT    360
   TTTATGACAC AAAGCGTCCA GGCCACCACA GAACTGACCG ATGCCTTAAT ATCAGCCTTT    420
   TCAGGTGTAT TACACGTTTC AACTGTAATC CCTCGCAATT GGGTAAACCG TCGGTGTGTA    480
   GGGATAAAGC GTAACCTTAC GTTCTGTCTC ATCTACAGGA TCATATTCAT CTGGGGAACC    540
   ATCCAGGACC ACGCGAATTC GCGTATCACC GGTCGCAGAA AACGGCAGAA ATAG          594
(2) INFORMATION ZU SEQ ID NO: 2:
   (i) SEQUENZ CHARAKTERISTIKA:
      (A) LÄNGE: 197 Aminosäuren
      (B) ART: Aminosäure
      (C) STRANGFORM: Einzel
      (D) TOPOLOGIE: linear
   (ii) ART DES MOLEKÜLS: Protein
   (v) ART DES FRAGMENTS: inneres
   (xi) SEQUENZBESCHREIBUNG: SEQ ID NO: 2:
   Met Ser Ser Thr Gln Ile Arg Thr Glu Ile Pro Val Ala Leu Leu Ile
    1               5                  10                  15
   Leu Cys Leu Cys Leu Val Ala Cys His Ala Asn Cys Pro Thr Tyr Arg
               20                  25                  30
   Ser His Leu Gly Phe Trp Gln Glu Gly Trp Ser Gly Gln Val Tyr Gln
           35                  40                  45
   Asp Trp Leu Gly Arg Met Asn Cys Ser Tyr Glu Asn Met Thr Ala Leu
       50                  55                  60
   Glu Ala Val Ser Leu Asn Gly Thr Arg Leu Ala Ala Gly Ser Pro Ser
   65                  70                  75                  80
   Ser Glu Tyr Pro Asn Val Ser Val Ser Val Glu Asp Thr Ser Ala Ser
                   85                  90                  95
   Gly Ser Gly Glu Asp Ala Ile Asp Glu Ser Gly Ser Gly Glu Glu Glu
               100                 105                 110
   Arg Pro Val Thr Ser His Val Thr Phe Met Thr Gln Ser Val Gln Ala
           115                 120                 125
   Thr Thr Glu Leu Thr Asp Ala Leu Ile Ser Ala Phe Ser Gly Val Leu
       130                 135                 140
   His Val Ser Thr Val Ile Pro Arg Asn Trp Val Asn Arg Arg Cys Val
   145                 150                 155                 160
   Gly Ile Lys Arg Asn Leu Thr Phe Cys Leu Ile Tyr Arg Ile Ile Phe
                   165                 170                 175
   Ile Trp Gly Thr Ile Gln Asp His Ala Asn Ser Arg Ile Thr Gly Arg
               180                 185                 190
   Arg Lys Arg Gln Lys
           195